(19) Europäisches Patentamt
European Patent Office
Office européen des brevets

(11) Publication number : **0 484 071 A2**

# (12) EUROPEAN PATENT APPLICATION

(21) Application number : **91309915.6**

(22) Date of filing : **28.10.91**

(51) Int. Cl.$^5$ : **A61K 31/70,** // (A61K31/70, 31:44)

(30) Priority : **01.11.90 US 608104**
**25.01.91 US 645857**

(43) Date of publication of application :
**06.05.92 Bulletin 92/19**

(84) Designated Contracting States :
**AT BE CH DE DK ES FR GB GR IT LI LU NL SE**

(71) Applicant : **MERCK & CO. INC.**
**126, East Lincoln Avenue P.O. Box 2000**
**Rahway New Jersey 07065-0900 (US)**

(72) Inventor : **Friedman, Paul A.**
**403 Great Spring Road**
**Rosemont, PA 19010 (US)**
Inventor : **Emini, Emilio A.**
**6 Faggs Manor Lane**
**Paoli, PA 19301 (US)**
Inventor : **Nunberg, Jack**
**167 Steepleschase Road**
**Devon, PA 19333 (US)**
Inventor : **Quintero, Julio C.**
**2700 Towamencin Avenue**
**Hatfield, PA 19440 (US)**

(74) Representative : **Thompson, John Dr. et al**
**Merck & Co., Inc. European Patent Department Terlings Park Eastwick Road**
**Harlow, Essex CM20 2QR (GB)**

## (54) Synergism of HIV reverse transcriptase inhibitors.

(57) The combination of certain aminopyridones and anti-HIV nucleoside analogs has been found to synergistically inhibit HIV reverse transcriptase. This combination is useful in the prevention or treatment of infection by HIV and the treatment of AIDS, either as a combination of compounds, pharmaceutically acceptable salts, pharmaceutical composition ingredients, whether or not in further combination with other antivirals, anti-infectives, immunomodulators, antibiotics or vaccines. Methods of treating AIDS and methods of preventing or treating infection by HIV are also described.

EP 0 484 071 A2

The present invention is concerned with a synergistic combination of compounds which inhibit the reverse transcriptase encoded by human immunodeficiency virus (HIV). This synergistic combination is of value in the prevention of infection by HIV, the treatment of infection by HIV and the treatment of the resulting acquired immune deficiency syndrome (AIDS). The present invention also relates to pharmaceutical compositions containing the synergistic combination and to a method of use of the present combination with or without other agents for the treatment of AIDS & viral infection by HIV.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is reverse transcription of the RNA genome by a virally encoded reverse transcriptase to generate DNA copies of HIV sequences, a required step in viral replication. It is known that some compounds are reverse transcriptase inhibitors and are effective agents in the treatment of AIDS and similar diseases, e.g., azidothymidine or AZT.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)].

Unexpectedly, applicants have found synergistic inhibition between two classes of structurally diverse compounds that inhibit HIV reverse transcriptase. The first class of compounds is {[(Benzoxazol-2-yl)methyl]amino}-5-alkyl-6-alkyl-2-(1H)-pyridones and related aminopyridones substituted with pyridyl or phenyl derivatives. The second class is active nucleoside analogs, such as AZT or ddI. Together these two classes offer superior therapy in the treatment of AIDS or HIV infection.

Further, the compounds of the first class are highly specific inhibitors of HIV reverse transcriptase, e.g., there is little or no inhibition of the reverse transcriptases of AMV, MMLV or SIV. Also, the compounds of the first class do not require bio-activation to be effective.

## BRIEF DESCRIPTION OF THE INVENTION

Synergistic combination of two classes of compounds, as herein defined, is disclosed. The combination is useful in the inhibition of HIV reverse transcriptase, the prevention of infection by HIV, the treatment of infection by HIV and in the treatment of AIDS and/or ARC, either as compounds, pharmaceutically acceptable salts (when appropriate), pharmaceutical composition ingredients, whether or not in combination with other antivirals, anti-infectives, immunomodulators, antibiotics or vaccines. Methods of treating AIDS, methods of preventing infection by HIV, and methods of treating infection by HIV are also disclosed.

## DETAILED DESCRIPTION OF THE INVENTION AND PREFERRED EMBODIMENTS

This invention is concerned with the combination of two classes of compounds in the inhibition of HIV reverse transcriptase, the prevention or treatment of infection by HIV and in the treatment of the resulting acquired immune deficiency syndrome (AIDS). The combination is defined as the use of a compound of Class A with a compound of Class B, wherein Class A consists essentially of;

3-{[(4,7-dichlorobenzoxazol-2-yl)methyl]amino}-5-ethyl-6-methyl-2-(1H)-pyridinone,
3{[(4,7-dimethylbenzoxazol-2-yl)methyl]amino}-5-ethyl-6-methyl-2-(1H)-pyridinone,
3-{[(7-chlorobenzoxazol-2-yl)methyl]amino}-5-ethyl-6-methyl-2-(1H)-pyridinone,
3-{[(7-methylbenzoxazol-2-yl)methyl]amino}-5-ethyl-6-methyl-2-(1H)-pyridinone,
3-{[(4-fluorobenzoxazol-2-yl)methyl]amino}-5-ethyl-6-methyl-2-(1H)-pyridinone,
3-{[(7-fluorobenzoxazol-2-yl)methyl]amino}-5-ethyl-6-methyl-2-(1H)-pyridinone,
3-{[(benzoxazol-2-yl)methyl]amino}-5-ethyl-6-methyl-2-(1H)-pyridinone,
3-{[(4-chlorobenzoxazol-2-yl)methyl]amino}-5-ethyl-6-methyl-2-(1H)-pyridinone,
3-{[(4-fluoro-7-chlorobenzoxazol-2-yl)methyl]amino}-5-ethyl-6-methyl-2-(1H)-pyridinone
3-[2-(benzoxazol-2-yl)ethyl]-5-ethyl-6-methyl-2-(1H)-pyridinone,
3-[N-(5-Ethyl-2-methoxy-6-methyl-3-pyridylmethyl)-amino]-5-ethyl-6-methyl-2(1H)-pyridinone,
3-[N-(5,6-Dimethyl-2-methoxy-3-pyridylmethyl)amino]-5-ethyl-6-methyl-2(1H)-pyridinone,
3-[N-(5-Ethyl-2-methoxybenzyl)amino]-5-ethyl-6-methyl-2(1H)-pyridinone,
3-[N-(2-Methoxy-4,5-dimethylbenzyl)amino]-5-ethyl-6-methyl-2(1H)-pyridinone, or

3-[N-(2,6-dimethoxybenzyl)amino]-5-ethyl-6-methyl-2(1H)-pyridinone;

Class B compounds consist essentially of;

3′-azido-2,3′-dideoxythymidine (also known as AZT)

2′,3′-dideoxycytidine (ddC)

2′,3′-dideoxyinosine (ddI)

2′,3′-didehydro-2′,3′-dideoxythymidine (D4T)

1-[(2-hydroxyethoxy)methyl]-6-phenyl-thio-thymine (HEPT), or

3′-fluoro-2′,3′-dideoxythymidine,

or pharmaceutically acceptable salt, hydrate or ester thereof.

One embodiment of the present invention is the combination of 3-{[(4,7-dichlorobenzoxazol-2-yl)-methyl]amino}-5-ethyl-6-methyl-2-(1H)-pyridinone, or 3{[(4,7-dimethylbenzoxazol-2-yl)methyl]amino}-5-ethyl-6-methyl-2-(1H)-pyridinone, with AZT, or ddI.

One preferred embodiment is the combination of 3-{[(4,7-dichlorobenzoxazol-2-yl)methyl]amino}-5-ethyl-6-methyl-2-(1H)-pyridinone, and AZT.

Another preferred embodiment is the combination of 3{[(4,7-dimethylbenzoxazol-2-yl)-methyl]amino}-5-ethyl-6-methyl-2-(1H)-pyridinone, and AZT.

A third preferred embodiment is the combination of 3{[(4,7-dimethylbenzoxazol-2-yl)-methyl]amino}-5-ethyl-6-methyl-2-(1H)-pyridinone, and ddI.

The compounds of the present invention may have asymmetric centers and occur as racemates, racemic mixtures and as individual diastereomers, with all isomeric forms being included in the present invention.

As used herein except where noted, "alkyl" is intended to include both branched- and straight-chain saturated aliphatic hydrocarbon groups having the specified number of carbon atoms.

Class A compounds are amino pyridones substituted at the 3 position with an amino alklene bridge bound to a heterocyclic or aryl moiety, such as benzoxazole. Class A compounds are highly potent inhibitors of HIV reverse transcriptase. In some cases, the bridge is alkylene.

In the synthesis of the class A compounds of this invention, the procedures and protocols of U.S. 3,721,676 can be followed for making many intermediates, which patent is incorporated by reference for these purposes. Applicants here provide preferred methods of synthesis, outlined below.

The 3-nitropyridone of the first step in the synthesis of 3 may be formed by a simultaneous condensation and cyclization of

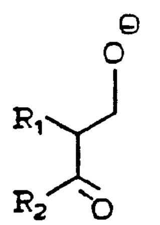

+

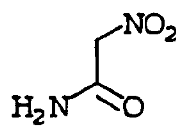

in the presence of a base such as piperidinium acetate. Nitroacetamide is prepared according to Brownstein, S.K., J. Org. Chem. 23, 113 (1958). The 3-nitropyridone products of the first step can also be prepared by direct nitration of 3-unsubstituted pyridones. The 3-nitropyridone product of the first step

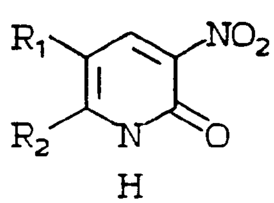

1

is reduced to

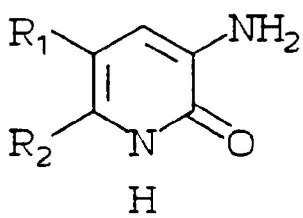

2

in a second step, preferably by catalytic reduction (when sulfur atoms are not present) in the presence of e.g. $H_2$ gas and Pd on carbon catalyst in a solvent such as ethanol. See, e.g., Okafor, C.O. et al., J. Heterocyclic Chem. 20, 199 (1983). Alternatively, the reduction of the second step (including when sulfur atoms are present) can be performed by chemical means, e.g. with NaSH, $Na_2S_2O_4$, Fe + $CaCl_2$, $H_2S$, or Sn + HCl. Reduction with iron in the presence of calcium chlorides is described in Okafor, C.O., J. Org. Chem. 47, 592 (1982). A third step, the final process giving rise to the compounds of this invention,

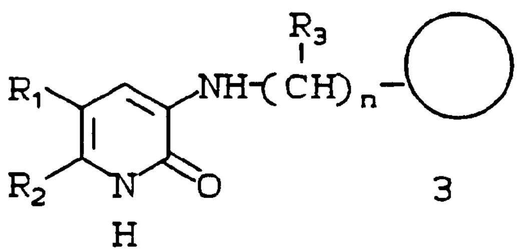

3

involves a coupling reaction, either by alkylation with an alkylhalide or a reductive alkylation with an aldehyde.

Carba derivatives (9), having alkylene bridges between the pyridone ring and the heterocyclic or aromatic entity attached at the 3-position, may be made by the following methods, the first being the preferred method.

In a first step 3-cyano-2-(1H) pyridinone is prepared by a simultaneous condensation and cyclization of

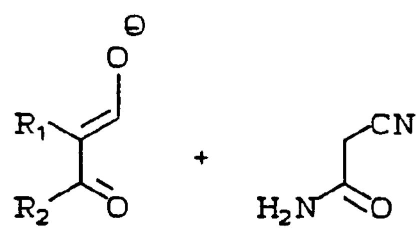

in the presence of base such as piperidinium acetate or pyrrolidine acetate. The resulting 3-cyano-2-(1H) pyridone,

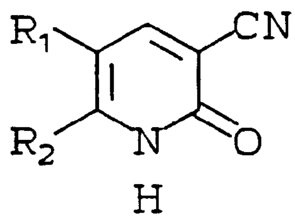

,

4

is heated in the presence of phosphorus pentachloride (sometimes in combination with $POCl_3$) to form the corresponding chloro pyridine,

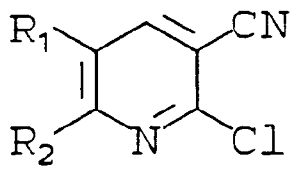

, 5

a conventional method of converting pyridones to chloropyridines. $PBr_3$ is useful for making the corresponding bromine derivative of product 5. Product 5 is then subjected to nucleophilic substitution to attach an alkoxy protecting group:

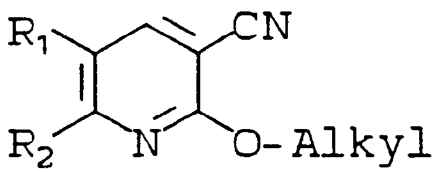

, 6

Reduction in the presence of, for example, diisobutyl aluminum hydride, yields

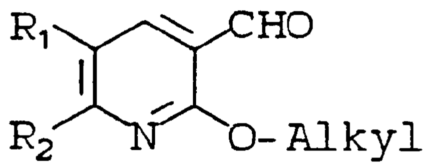

, 7

which is a conventional method of reducing nitriles to aldehydes. An alternative reducing agent for the synthesis of 7 is lithium trialkoxy aluminum hydride.

Condensation in the presence of base at very low temperature (preferably at least -100°C) with an alkyl-substituted aryl or alkyl substituted heterocycle yields

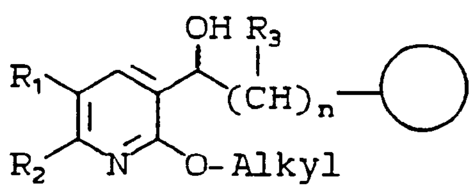

8

(n = 1).

(The Wittig approach, infra, is preferable when n is more than 1.) Dehydration of the alcohol, and dealkylation followed by hydrogenation results in the compounds of the invention, wherein X = $CH_2$,

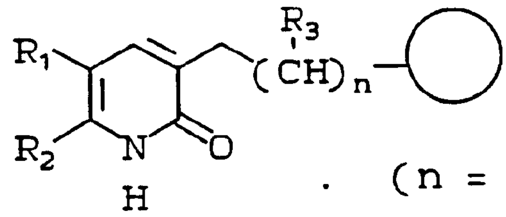

9

. (n = 1)

The dealkylation is typically carried out with pyridine hydrochloride in the presence of heat. The dehydration need not occur in the same reaction, but may be performed separately by conventional means. The standard catalyst for hydrogenation to produce product 9 is palladium, but other noble metals may substitute. Alternatively, the hydrogenation can be carried out with diimide or Raney nickel. $BCl_3$ or $BBr_3$ at low temperature is useful for performing dealkylation without dehydration.

A second alternative variation on the final 3 steps is the Wittig approach, wherein the aldehyde derivative (product 7 above) is reacted with aryl or heterocycle, each substituted with alkylene triphenylphosphorane. The resulting condensation yields an unsaturated alkylene bridge, which is then hydrogenated to yield product 9 compounds above. The advantage of the Wittig approach is that it is preferable for synthesis of compounds of formula 9 wherein n is 2 or more, i.e. with longer alkylene bridges. Also, the Wittig approach is preferable for compounds of Class A I having labile substituents on the heterocycle, particularly halogens.

Class B compounds are nucleoside analogs having known biological activity against HIV reverse transcriptase. They are in general chain terminators. A separate synthesis is provided for each compound.

AZT is synthesized by the methods of J.P. Horwitz et al., J. Org. Chem. 29, 2076 (1964); R.P. Glinski et al., J. Org. Chem. 38, 4299 (1973); C.K. Chu et al., Tetrahedron Letters 29, 5349 (1988), each incorporated by reference for this purpose. Application of AZT as a therapeutic drug in the treatment of AIDS is disclosed in U.S. 4,724,232.

The compound ddC is synthesized by the methods of J.P. Horwitz et al., J. Org. Chem. 32, 817 (1967); R. Marumoto, Chem. Pharm. Bull. 22, 128 (1974); and T.-S. Lin et al., J. Med. Chem. 30, 440(1987), each incorporated by reference for this purpose.

D4T is synthesized by the methods of Herdewijn, P. et al., J. Med. Chem. 30, 1270 (1987), herein incorporated by reference for this purpose.

HEPT is synthesized by the methods of Miyasaka, T. et. al. J, Med, Chem, 32, 2507 (1989); and A. Rosowsky, J. Med. Chem. 24, 1177 (1981), each incorporated by reference for this purpose.

The compound 3′-fluoro-2′,3′-dideoxythymidine is synthesized by the procedures of Herdewijn, P. et al., J. Med, Chem, 30, 1270 (1987), herein incorporated by reference for this purpose.

The synergistic combination of the present invention is useful in the inhibition of HIV reverse transcriptase, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the combinations of this invention are useful in treating infection by HIV after suspected past exposure to HIV by, e.g., blood transfusion, accidental needle stick, or exposure to patient blood during surgery.

For these purposes, the compounds of the present invention are administered in combination, either orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a combination of therapeutically-effective amounts of the compounds of the present invention.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients,

binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquify and/or dissolve in the rectal cavity to release the drug.

The compounds of this invention can be administered orally to humans in a dosage range of 1 mg to 5 g/kg body weight in divided doses. A preferred dosage range is 1-100 mg/kg body weight for Class A compounds administered orally in divided doses, and 50 mg to 5 g/kg body weight for Class B compounds adminstered orally in divided doses. It will be understood, however, that the specific dose level, dosage ratio, and frequency of dosage for any particular patient may be varied and will depend upon a variety of factors including the activity or potency of the specific compound employed, the metabolic stability and length of action of that compound, the age of the patient, body weight, general health, sex, diet, mode and time of administration, rate of excretion, drug combination, the severity of the particular condition, and the host undergoing therapy.

The present invention is also directed to combinations of the HIV reverse transcriptase inhibitor compounds with one or more agents useful in the treatment of AIDS. For example, the compounds of this invention may be effectively administered, whether at periods of pre-exposure and/or post-exposure, in combination with effective amounts of other AIDS antivirals, immunomodulators, anti-infectives, or vaccines.

EXAMPLE 1

Preparation of 5-ethyl-6-methyl-3-(2-napthylmethylamino)-2-(1H)-pyridinone

Step A: Preparation of 5-ethyl-6-methyl-3-nitro-2-(1H)-pyridinone

A mixture of 2-ethyl-3-oxobutanal, sodium salt (7.5 g, 55 mmol), nitroacetamide (6.6 g, 63 mmol), aqueous piperidinium acetate (4.4 mL) [prepared from glacial acetic acid (42 mL), water (100 mL) and piperidine (72 mL)] in water (45 mL) was stirred at room temperature for 22 hours. The yellow precipitate was collected by filtration and air dried to yield 8.0 g (80%) of 5-ethyl-6-methyl-3-nitro-2-(1H)-pyidinone.

Step B: Preparation of 3-amino-5-ethyl-6-methyl-2-(1H)-pyridinone

A yellow solution of the 5-ethyl-6-methyl-3-nitro-2-(1H)-pyidinone (10 g, 55 mmol) in a mixture of methanol and tetrahydrofuran (100 mL, 1:1 v/v) was reduced catalytically in the presence of 7% palladium on charcoal (0.7 g ) under an atmosphere of hydrogen (50 psi) at room temperature over a period of 3.5 hours. The resultant mixture was filtered through a small pad of Celite. The filtrate was concentrated under reduced pressure (15 torr) to provide 5.7 g (68%) of the corresponding aminopyridone.

Step C: Preparation of 5-ethyl-6-methyl-3-(2-napthyl-methylamino)-2-(1H)-pyridinone

A mixture of 3-amino-5-ethyl-6-methyl-2-(1H)-pyridinone (3.0 g, 20 mmol), 2-bromomethylnapthalene (4.4 g, 20 mmol), triethylamine (2.0 g, 20 mmol) in acetonitrile (200 mL) was heated under reflux for 4 hours. The resultant mixture was allowed to cool to room temperature and concentrated under reduced pressure (15 torr). The residue was then subjected to column chromatography on silica gel (300 g, elution with methanol-chloroform, 5:95 v/v). Collection and concentration of appropriate fractions provided 2.4 g (42%) of the napthylmethylaminopyridone.

Anal. Calcd for $C_{19}H_{20}N_2O$:
C, 75.71; H, 7.02; N, 9.30.
Found: C, 75.73; H, 6.71; N, 9.13.

EXAMPLE 2

3-[(2-Benzoxazolylmethyl)amino]-5-ethyl-6-methyl-2-(1H)-pyridinone

A solution of 3-amino-5-ethyl-6-methyl-2-(1H)-pyridinone (152 mg,1.0 mmol), 2-chloromethyl-1,3-benzoxazole (1.07 mmol) and triethylamine (0.14 mL, 1.0 mmol) in acetonitrile (10 mmL) was stirred at reflux for 24 hrs. After concentrating under reduced pressure,the residue was flash chromatographed over silica gel. Elution with 5% MeOH- 95% $CHCl_3$ gave 132 mg of product which was recrystallized from EtOH-water to give 95 mg of analytically pure product, mp 202-203°C, with initial melting at 179° followed by resolidification, Anal. Calcd for $C_{16}H_{17}N_3O_2$:

C, 67.83, H, 6.05; N, 14.83

Found: C, 67.71; H, 6.03; N, 14.76.

EXAMPLE 3

Preparation of 3-[((4,7-Dimethylbenzoxazol-2-yl)-methyl)amino]-5-ethyl-6-methyl-2(1H)-pyridinone

Step A: Preparation of 2-chloromethyl-4,7-dimethyl-benzoxazole

To a solution of 2,5-dimethyl-6-aminophenol (0.67 g, 4.9 mmol) in methylene chloride, solid ethyl 2-chloroiminoacetate hydrochloride (0.85 g, 4.9 mmol) was added. The resultant slurry was stirred at room temperature for 18 hours, then filtered through a plug of diatomaceous earth and concentrated under reduced pressure (15 torr). The solid residue was subjected to column chromatography on silica gel (50 g, eluted with 1% methanol in chloroform).
Collection and concentration of appropriate fractions yielded 0.85 g (89%) of the benzoxazole.

Step B: Preparation of 3-[((4,7-dimethylbenzoxazol-2-yl)methyl)amino]-5-ethyl-6-methyl-2-(1H)-pyridinone

A mixture of 3-amino-5-ethyl-6-methyl-2-(1H)-pyridinone (0.23 g, 1.5 mmol), 2-chloromethyl-4,7-dimethylbenzoxazole (0.29 g, 1.5 mmol), diisopropylethylamine (0.39 g, 3 mmol) in acetonitrile (50 mL) was refluxed under an atmosphere of nitrogen for 12 hours. The resultant mixture was concentrated under reduced pressure (15 torr). The residue was then subjected to column chromatography on silica gel (100 g, elution with 4% methanol in chloroform).
Collection and concentration of appropriate fractions provided 0.2 g (44%) of the benzoxazolylmethylaminopyridone. Anal. Calcd for $C_{18}H_{21}N_3O_2$:

C, 69.43; H, 6.80; N, 13.49.

Found: C, 69.32; H, 6.66; N, 13.47.

EXAMPLE 4

Preparation of 3-[2-(benzoxazol-2-yl)ethyl]-5-ethyl-6-methyl-2-(1H)-pyridinone

Step A: Preparation of 3-cyano-5-ethyl-6-methyl-2-(1H)-pyridinone

Accordingly to the method described in J. Heterocyclic Chem., 24, 351 (1987), a mixture 2-ethyl-3-oxobutanol, sodium salt (37.5 g, 0.275 mol), cyanoacetamide (25.2 g, 0.30 mol), aqueous piperidinium acetate (22 mL) [prepared from glacial acetic acid (4.2 mL), water (10 mL) and piperidine (7.2 mL)] in water (775 ml) was refluxed for four hours. Glacial acetic acid (30 ml) was added cautiously (much foaming) as the product precipitated. Upon cooling to room temperature, the product was collected by filtration, washed with cold water and air dried to yield 22.3 g (50%), m.p. 237-240°C.

Step B: Preparation of 2-chloro-3-cyano-5-ethyl-6-methylpyridine

3-Cyano-5-ethyl-6-methyl-2-(1H)-pyridinone (22.9 g, .141 mol) and phosphorus pentachloride (33.1

g, .159 mol) were intimately mixed and heated at 110-120°C for one hour. The liquified solids were poured onto crushed ice and water and the semi-solid was extracted into chloroform. This extract was washed with water, saturated aqueous $NaHCO_3$, dried ($Na_2SO_4$), filtered and evaporated. This amber oil was dissolved in hexane and the insoluble material was removed when filtered through a pad of charcoal. Removal of the solvent gave a light yellow oil which solidified (17.7 g). Trituration of this solid with cold hexane yielded 15.6 g (61%) of pure product, m.p. 63-64°C.

Step C: Preparation of 2-methoxy-3-cyano-5-ethyl-6-methylpyridine

Sodium metal (3.25 g, .141 mol) was dissolved in dry methanol (100 mL) under a nitrogen atmosphere. When solution was complete, a slurry of 2-chloro-5-ethyl-6-methylpyridine (17.95 g, 99.4 mmol) in dry methanol (70 mL) was added and the reaction was warmed at 60°C for 15-20 hours. After cooling the reaction mixture, diethyl ether (250 mL) and water (200 mL) were added. The ether layer was separated and washed with water, dried ($Na_2SO_4$), filtered and evaporated to give a light yellow solid (17.5 g). This solid was triturated with cold hexane to yield 14.4 g (82%) of pure product, m.p. 59-61°C.

Step D: Preparation of 2-methoxy-5-ethyl-6-methyl-nicotinaldehyde

To a solution of 2-methoxy-3-cyano-5-ethyl-6-methylpyridine (1.0 g, 5.68 mmol) in dry tetrahydrofuran (50 mL) under a nitrogen atmosphere and cooled to -70°C, was added 1.3M diisobutyl aluminum hydride/THF (17.4 mL, 22.7 mmol). The resulting mixture was allowed to warm to room temperature and stir for 15-20 hours. The reaction mixture was acidified with 1N hydrochloric acid and then neutralized with aqueous sodium bicarbonate. Water was then added and the product extracted into diethyl ether. The etheral extract was dried ($Na_2SO_4$), filtered and the solvent evaporated. This residue was flash chromatographed on silica gel eluting with 10% diethyl ether/pentane to give 610 mg (61%) of product.

Step E: Preparation of 2-[2(R/S)-hydroxy-2-(2-methoxy-5-ethyl-6-methylpyridin-3-yl)ethyl]-benzoxazole

To a solution of 2-methylbenzoxazole (226 mg, 1.7 mmol) in anhydrous THF (4 mL), cooled to -100°C under an argon atmosphere, was added 1.6M n-butyl-lithium/hexane (1.05 mL) slowly over 35 minutes. After 0.5 hour a solution of 2-methoxy-5-ethyl-6-methylnicotinaldehyde (300 mg, 1.7 mmol) in dry THF (1 mL) was added dropwise. The reaction was allowed to warm to room temperature and poured onto crushed ice. This mixture was extracted with diethyl ether. The combined extracts were dried ($MgSO_4$) and the solvent removed to give an oil which was flash chromatographed over silica gel. Elution with ethyl acetate/hexane (1:19) gave 340 mg (65%) of analytically pure racemic product, mp 102-103°C. Anal. Calcd for $C_{18}H_{20}N_2O_3 \cdot 0.1\ H_2O$:

C, 68.81; H, 6.48; N, 8.92.
Found: C, 68.80; H, 6.76; N, 8.95.

Step F: Preparation of 3-[2-(benzoxazol-2-yl)-ethenyl]-5-ethyl-6-methyl-2-(1H)-pyridinone

A mixture of 2-[2(R/S)-hydroxy-2-(2-methoxy-5-ethyl-6-methylpyridin-3-yl)ethyl]benzoxazole (72 mg, 0.23 mmol) and pyridine hydrochloride (133 mg, 1.2 mmol), under a nitrogen atmosphere, was placed in a preheated oil bath (165°C) for 5 minutes. The reaction flask was removed, cooled, and water added to give a solid. This crude product was extracted into chloroform, dried ($MgSO_4$) and the solvent evaporated to yield 49 mg (75%) of pure product. Recrystallization from methanol gave 15 mg of analytically pure product, mp 262-264°C. Anal. Calcd for $C_{17}H_{16}N_2O_2$:

C, 72.83; H, 5.75; N, 10.00.
Found: C, 72.93; H, 5.95; N, 9.99.

Step G: Preparation of 3-[2-(benzoxazol-2-yl)ethyl]-5-ethyl-6-methyl-2-(1H)-pyridinone

A solution of 80% pure 3-[2-(benzoxazol-2-yl)ethenyl]-5-ethyl-6-methyl-2-(1H)-pyridinone (200 mg) in methanol/ethanol/THF (25 mL, 1:1:1) was hydrogenated at atmospheric pressure over 5% palladium/charcoal for four hours. After filtering off the catalyst, the solvents were evaporated and the residue flash

chromatographed over silica gel. Elution with 2% methanol-98% chloroform gave 75 mg of analytically pure product, mp 155-156.5°C. Anal. Calcd. for $C_{17}H_{18}N_2O_2$

C, 72.31; H, 6.43; N, 9.92.
Found: C, 72.45; H, 6.52; N, 9.99.

EXAMPLE 5

Preparation of 3-[2-(4,7-dichlorobenzoxazolyl)methyl]-amino-5-ethyl-6-methyl-1H-pyridin-2-one

Step A: Preparation of 2-amino-3,6-dichlorophenol

A yellow solution of 2,5-dichloro-6-nitrophenol (10.0 g 48.0 mmol) in ethanol (200 mL) and acetic acid (13.8 mL) at 0°C was catalytically reduced in the presence of 5% Platinum on charcoal (0.15 g) under an atmosphere of hydrogen (25 psi) for 1 hour in a Parr hydrogenator. The resultant colorless solution was filtered and concentrated under reduced pressure (15 torr). The residue was then dried under high vacuum (0.02 torr) overnight to yield 8.52 g (100%) of 2-amino-3,6-dichlorophenol.

Step B: Preparation of 2-chloromethyl-4,7-dichloro-benzoxazole

To a solution of 2-amino-3,6-dichlorophenol (23.91 g, 134 mmol) in methylene chloride (270 mL), solid ethyl chloroiminoacetate hydrogen chloride (31.9 g, 202 mmol) was added. The resultant slurry was stirred at room temperature overnight, then filtered through a plug of Celite, and concentrated under reduced pressure (15 torr). The solid residue was subjected to column chromatography on silica gel (elution with chloroform). Collection and concentration of appropriate fractions yielded 26.6 g (86%) of 2-chloromethyl-4,7-dichlorobenzoxazole.

Step C: Preparation of 3-[2-(4,7-dichlorobenzoxazolyl)methyl]amino-5-ethyl-6-methyl-1H-pyridin-2-one

A mixture of 3-amino-5-ethyl-6-methylpyridine-2-one (0.93 g, 6.1 mmol), 2-chloromethyl-4,7-dichlorobenzoxazole (1.45 g, 6.1 mmol), diisopropylethylamine (1.06 mL, 6.1 mmol) in acetonitrile (30 mL) was reluxed under an atmosphere of nitrogen for 20 hours. The resultant mixture was cooled to 0°C. The solide precipitated was filtered and subjected to column chromatography on silica gel (elution with 4% methanol in chloroform). Collection and concentration of appropriate fractions provided 0.76 g of a white solid which was then recrystallized from ethanol to yield 0.66 g (31%) of 3-[2-(4,7-dichlorobenzoxazoly) methyl]amino-5-ethyl-6-methyl-1H-pyridin-2-one. Anal. Calcd, for $C_{16}H_{15}C_{12}N_3OS$:

C, 54.56; H, 4.29; N, 11.93
Found C, 54.43; H, 4.12; N, 1.89%

EXAMPLE 6

INHIBITION OF VIRUS SPREAD

A. Preparation of HIV-infected MT-4 cell Suspension.

MT cells were infected at Day 0 at a concentration of 250,000 per ml with a 1:2000 dilution of HIV-1 strain IIIb stock (final 125 pg p24/ml; sufficient to yield ≤1% infected cells on day 1 and 25-100% on day 4). Cells were infected and grown in the following medium: RPMI 1640 (Whittaker BioProducts), 10% inactivated fetal bovine serum, 4 mM glutamine (Gibco Labs) and 1:100 Penicillin-Streptomycin (Gibco Labs).

The mixture was incubated overnight at 37°C in 5% $CO_2$ atmosphere.

B. Treatment with Inhibitors

A matrix of nanomolar range concentration of 661 [3-{[(4,7-dichlorobenzoxazol-2-yl)methyl]amino} amino}-5-ethyl-6-methyl-2-(1H)-pyridinone] and AZT was prepared. At Day 1, aliquots of 125 μl of inhibitors were added to equal volumes of HIV-infected MT-4 cells (50,000 per well) in a 96-well microtiter cell culture plate. Incubation

was continued for 3 days at 37°C in 5% $CO_2$ atmosphere.

C. Measurement of Virus Spread

Using a multichannel pipettor, the settled cells were resuspended and 125 µl harvested into a separate microtiter plate. After the settling of the cells, the plates were frozen for subsequent assay of the supernatant for HIV p24 antigen.

The concentration of HIV p24 antigen was measured by an enzyme immunoassay, described as follows. Aliquots of p24 antigen to be measured were added to microwells coated with a monoclonal antibody specific for HIV core antigen. The microwells were washed at this point, and at other appropriate steps that follow. Biotinylated HIV-specific antibody was then added, followed by conjugated streptavidin-horseradish peroxidase. A color reaction occurs from the added hydrogen peroxide and tetramethylbenzidine substrate. Color intensity is proportional to the concentration of HIV p24 antigen.

## EXAMPLE 7

### Calculation of Degree of Synergy

Combinations of 661 (defined in Example 6B) and AZT (see Example 6) were found to exhibit markedly enhanced inhibition of virus spread, in comparison to 661 or AZT alone, or in comparison to merely additive inhibition of 661 and AZT.

This data was processed in two separate ways:

(1) fractional inhibitory concentration ratios (FIC) were calculated according to Elion, et. al. J. Biol. Chem., 208, 477 (1954). The minimum sum of FICS, which is the maximum synergy, was found to be about 0.4 for 661 and AZT.

(2) Synergism was also evaluated according to the method of Chou, T.-S. et al., "Applications of the Median-Effect Principle for the Assessment of Low-Dose Risk of Carcinogens and for the Quantitation of Synergism and Antagonism of Chemotherapeutic Agents," in Harrap, K.R. et. al. (eds.), New Avenues in Developmental Cancer Chemotherapy (1987), page 37 et seq. Comparable results were obtained.

## EXAMPLE 8

### Preparation of 3-[N-(5-ethyl-2-methoxy-6-methyl-3-pyridylmethyl)amino]-5-ethyl-6-methyl-2(1H)-pyridinone

A solution of 3-amino-5-ethyl-6-methyl-2(1H)-pyridinone (12.66 g, 83.2 mmol), 5-ethyl-2-methoxy-6-methylnicotinaldehyde (15.0 g, 83.2 mmol), and acetic acid (5 drops in methanol (83 mL) was stirred at room temperature for 18 hours under an atmosphere of nitrogen. The yellow-orange precipitate was filtered, washed with a small amount of methanol, and then dissolved in a mixture of methanol and chloroform (3:1 v/v) with warming. The resultant solution was allowed to cool back to room temperature and sodium cyanoborohydride was added until all the Schiff base was reduced. The product solution was then concentrated under vacuum. Water and chloroform were added to the residue. The aqueous layer was separated and extracted three more times with chloroform. The organic extracts were combined, dried over anhydrous sodium sulfate, filtered and concentrated under vacuum. The residue was then subjected to column chromatography on silica gel and eluted with 5% methanol in chloroform. Collection and concentration of appropriate fraction, followed by recrystallization (ethanol) yielded 10.5 g (40%) of the title compound. Anal. Calcd. for $C_{18}H_{25}N_3O_2$:

| | |
|---|---|
| | C, 68.54; H, 7.99; N, 13.32. |
| Found | C, 69.17; H, 7.99; N, 13.36%. |

## EXAMPLE 9

### 3-[N-(2,6-Dimethoxybenzyl)amino]-5-ethyl-6-methyl-2(1H)-pyridinone

3-Amino-5-ethyl-6-methyl-2(1H)-pyridinone (152 mg, 1.00 mmol) and 2,6-dimethoxybenzaldehyde (168 mg, 1.00 mmol) was dissolved in methanol (5 mL). Two drops of glacial acetic acid were added and the solution stirred for 2 hours. Sodium borohydride (378 mg, 10 mmol) was added portionwise to the suspension and the reaction stirred for 30 minutes at room temperature. The mixture was diluted with water and extracted with ethyl

acetate. The ethyl acetate was washed with water, saturated brine, and dried over magnesium sulfate. The drying agent was filtered and the filtrate concentrated in vacuo to give a white solid (290 mg, 96% yield). This solid was recrystallized from ethanol to give the title compound as white fluffy needles (140 mg) mp 220-222°C.

Anal. Calcd. for $C_{17}H_{22}N_2O_3$:
C, 67.52; H, 7.34; N, 9.26.
Found C, 67.69; H, 7.50; N, 9.18.

$^1H$ NMR (DMSO-$d_6$, 300 MHz):δ 11.1 (1H, s), 7.20 (1H, t, J=8 Hz), 6.65 (2H, d, J=8 Hz), 6.30 (1H, s) 4.80 (1H, s), 4.16 (2H, s), 3.80 (6H, s) 2.26 (2H, q, J=7 Hz), 2.00 (3H, s), 1.00 (3H, t, J=7 Hz).

EXAMPLE 10

3-[N-(5-Ethyl-2-methoxybenzyl)amino]-5-ethyl-6-methyl-2(1H)-pyridinone

Step A: 4-Ethylanisole

To a suspension of powdered KOH (9.18 g, 0.16 mol) in DMSO (80 mL) was added 4-ethylphenol (500 g, 0.041 mol) followed by iodomethane (5.0 mL, 0.081 mol). The reaction was stirred for 10 minutes, poured into water (800 mL), and extracted with benzene (3 X 200 mL). The organic extracts were washed with water, saturated brine and dried over sodium sulfate. Filtration and concentration of the filtrate in vacuo gave the crude product (6.21 g) which was chromatographed on silica gel with 5% ethyl acetate in hexane. The title compound was obtained as a clear oil (4.67 g, 84% yield).
$^1H$ NMR ($CDCl_3$, 300 MHz): δ 7.38 (1H, s), 7.11 (1H, d, J=7 Hz), 6.83 (1H, d, J=7 Hz), 3.79 (3H, s), 3.58 (2H, q, J=7 Hz), 1.21 (3H, t, J=7 Hz).

Step B: 4-Ethyl-2-methoxypenzaldehyde

A solution of 4-ethylanisole (4.54 g, 0.033 mol) in dry ether (45 mL) was cooled to 0°C under a nitrogen atmoshpere. To this solution was added n-butyllithium (13.3 mL of a 2.5 M solution in hexane) via syringe and the reaction stirred at room temperature overnight. The reaction was cooled to -78°C and DMF (5.17 mL, 0.066 mol) (dry, distilled) was added. The reaction was stirred at -78°C for 5 minutes, 0°C for 25 minutes and then 10% HCL added. The layers were separated and the organic phase washed with water, saturated brine and dried over sodium sulfate. The crude product was obtained as a oil (5.56 g). Of this material 4.35 g was chromatographed on silica gel with 5% ethyl acetate in hexane. The title compound was obtained as a clear oil (2.18 g, 51% yield).
$^1H$ NMR ($CDCl_3$, 300 MHz): δ 10.45 (1H, s), 7.66 (1H, d, J=2 Hz), 7.39 (1H, dd, J=2, 8 Hz), 6.92 (1H, d, J=8 Hz), 3.91 (3H, s), 2.62 (2H, q, J=7 Hz), 1.22 (3H, t, J=7 Hz).

Step C. 3-[N-(5-Ethyl-2-methoxybenzyl)amino]-5-ethyl-6-methyl-2(1H)-pyridinone

4-Ethyl-2-methoxybenzaldehyde (0.561 g, 3.42 mmol) 3-amino-5-ethyl-6-methyl-2(1H)-pyridinone (0.520 g, 3.42 mmol) were reacted according to the procedure described for the preparation of 3-[N-(2,6-dimethoxybenxyl)]-amino-5-ethyl-6-methyl-2(1H)-pyridinone. The crude product was recrystallized from ethanol to give the title compound as pale yellow prisms, mp 149-152°C (0.370 g).

Anal. Calcd. for $C_{18}H_{24}N_2O_2$:
C, 71.71; H, 8.05; N, 9.32.
Found C, 71.93; H, 8.05; N, 9.18.

$^1H$ NMR ($CDCl_3$, 300 MHz): δ 11.33 (1H, br s), 7.13 (1H, d, J=2 Hz), 7.06 (1H, dd, J=2, 8 Hz), 6.80 (1H, d, J=8 Hz), 6.21 (1H, s), 5.08 (1H, br, m), 4.28 (2H, d, J=6 Hz), 3.83 (3H, s) 2.55 (2H, q, J=7 Hz), 2.32 (2H, q J=7 Hz), 2.19 (3H, s), 1.16 (3H, t, J=7 Hz), 1.05 (3H, t, J=7 Hz).

EXAMPLE 11

3-[N-(2-methoxy-4,5-dimethylbenzyl)amino]-5-ethyl-6-methyl-2(1H)-pyridinone

Step A. 4,5-Dimethyl-2-methoxybenzaldehyde

A solution of 3,4-dimethylanisole (9.74 g, 0.071 mol) in dry ether (150 mL) was cooled under nitrogen to 0°C. To this stirred solution was added n-butyllithium (28.6 mL 2.5 M in hexane) via syringe. The reaction was stirred at 0°C for 1.5 hours then at room temperature for 16 hours. The reaction was cooled to 0°C and DMF (11.0 mL, 2 eq.) was added. The reaction was stirred at 0°C for 1.5 hours, diluted with 10% aqueous HCl and the layers separated. The ether solution was washed with water, saturated brine, and dried over magnesium sulfate. The drying agent was filtered and the solvent removed in vacuo to give two products $R_f$ = 0.37 and $R_f$ = 0.25 with 5% ethyl acetate in hexane on a silica gel tlc plate. The crude products were separated on silica gel with 5% ethyl acetate in hexane. The $R_f$ = 0.37 material was isolated as a clear oil which solidified on standing and was identified as 5,6-dimethyl-2-methoxybenzaldehyde (1.4 g, 11% yield). The $R_f$ = 0.25 material was the title compound and was obtained as a white solid, mp 63-66°C (3.6 g, 30% yield)
$^1H$ NMR (300 MHz, $CDCl_3$): δ 10.38 (1H, s), 7.57 (1H, s), 6.76 (1H, s), 3.89 (3H, s), 2.32 (3H, s), 2.22 (3H, s).

Step B. 3-[N-(2-methoxy-4,5-dimethylbenzyl)amino]-5-ethyl-6-methyl-2(1H)-pyridinone

4,5-dimethyl-2-methoxybenzaldehyde (240 mg, 1.45 mmol) and 3-amino-5-ethyl-6-methyl-2(1H)-pyridinone (220 mg, 1.45 mmol) were reacted according to the procedure described for the preparation of 3-[N-(2,6-dimethoxybenzyl)]-amino-5-ethyl-6-methyl-2(1H)-pyridinone. The crude product (426 mg, 97% yield) was recrystallized from methanol (25 mL) to give the title compound as a pale yellow solid (273 mg) mp 175.5-177°C.
$^1H$ NMR (300 MHz, DMSO-$d_6$): δ 11.1 (1H, br, s), 6.97 (1H, s), 6.78 (1H, s), 6.08 (1H, s), 5.3 (br, s, 1H), 4.10 (2H, s), 3.77 (3H, s), 2.22 (2H, q, J=7.6 Hz), 2.18 (3H, s), 2.09 (3H,s), 2.01 (3H, s) 0.97 (3H, t, J=7.5 Hz).

While the foregoing specification teaches the principles of the present invention, with examples provided for the purpose of illustration, it will be understood that the practice of the invention encompasses all of the usual variations, adaptations, and modifications, as come within the scope of the following claims and its equivalents.

## Claims

1. A synergistic combination of a compound of Class A and a compound of Class B, wherein Class A consists essentially of:
3-{[(4,7-dichlorobenzoaxazol-2-yl)methyl]amino}-5-ethyl-6-methyl-2-(1H)-pyridinone,
3{[(4,7-dimethylbenzoxazol-2-yl)methyl]amino}-5-ethyl-6-methyl-2-(1H)-pyridinone,
3-{[(7-chlorobenzoxazol-2-yl)methyl]amino}-5-ethyl-6-methyl-2-(1H)-pyridinone,
3-{[(7-methylbenzoxazol-2-yl)methyl]amino}-5-ethyl-6-methyl-2-(1H)-pyridinone,
3-{[(4-fluorobenzoxazol-2-yl)methyl]amino}-5-ethyl-6-methyl-2-(1H)-pyridinone,
3-{[(7-fluorobenzoxazol-2-yl)methyl]amino}-5-ethyl-6-methyl-2-(1H)-pyridinone,
3-{[(benzoxazol-2-yl)methyl]amino}-5-ethyl-6-methyl-2-(1H)-pyridinone,
3-{[(4-chlorobenzoxazol-2-yl)methyl]amino}-5-ethyl-6-methyl-2-(1H)-pyridinone,
3-{[(4-fluoro-7-chlorobenzoxazol-2-yl)methyl]amino}-5-ethyl-6-methyl-2-(1H)-pyridinone,
3-[2-(benzoxazol-2-yl)ethyl]-5-ethyl-6-methyl-2-(1H)-pyridinone,
3-[N-(5-Ethyl-2-methoxy-6-methyl-3-pyridylmethyl)-amino]-5-ethyl-6-methyl-2(1H)-pyridinone,
3-[N-(5,6-Dimethyl-2-methoxy-3-pyridylmethyl)amino]-5-ethyl-6-methyl-2(1H)-pyridinone,
3-[N-(5-Ethyl-2-methoxybenzyl)amino]-5-ethyl-6-methyl-2(1H)-pyridinone,
3-[N-(2-Methoxy-4,5-dimethylbenzyl)amino]-5-ethyl-6-methyl-2(1H)-pyridinone, or
3-[N-(2,6-dimethoxybenzyl)amino]-5-ethyl-6-methyl-2(1H)-pyridinone;
Class B consists essentially of:
3′-azido-2,3′-dideoxythymidine;
2′,3′-dideoxycytidine;
2′,3′-dideoxyinosine;
2′,3′-didehydro-2′,3′-dideoxythymidine;
1-[(2-hydroxyethoxy)methyl]-6-phenyl-thiothymine; or
3′-fluoro-2′,3′-dideoxythymidine,

or pharmaceutically acceptable salt, hydrate or ester thereof.

2. The combination of Claim 1 wherein Class A consists essentially of:
3-{[(4,7-dichlorobenzoxazol-2-yl)methyl]-amino}-5-ethyl-6-methyl-2-(1H)-pyridinone, or
3{[(4,7-dimethylbenzoxazol-2-yl)methyl]-amino}-5-ethyl-6-methyl-2-(1H)-pyridinone;
Class B consists essentially of:
3′-azido-2′,3′-dideoxythymidine, or 2′,3′-dideoxyinosine,
or pharmaceutically acceptable salt thereof.

3. The combination of Claim 1 wherein Class A consists essentially of:
3-{[(4,7-dichlorobenzoxazol-2-yl)methyl]amino}-5-ethyl-6-methyl-2-(1H)-pyridinone;
Class B consists essentially of:
3′-azido-2,3′-dideoxythymidine, or pharmaceutically acceptable salt, hydrate or ester thereof.

4. The combination of Claim 1 wherein Class A consists essentially of:
3{[(4,7-dimethylbenzoxazol-2-yl)methyl]-amino}-5-ethyl-6-methyl-2-(1H)-pyridinone;
Class B consists essentially of:
3′-azido-2,3′-dideoxythymidine, or pharmaceutically acceptable salt, hydrate or ester thereof.

5. The combination of Claim 1 wherein Class A consists essentially of:
3{[(4,7-dimethylbenzoxazol-2-yl)methyl]-amino}-5-ethyl-6-methyl-2-(1H)-pyridinone;
Class B consists essentially of:
2′3′-dideoxyinosine,
or pharmaceutically acceptable salt, hydrate or ester thereof.

6. The use of the combination as claimed in any one of Claims 1 to 5 for the manufacture of a medicament for synergistically inhibiting HIV reverse transcriptase.

7. The use of the combination as claimed in any one of Claims 1 to 5 for the manufacture of a medicament for preventing infection by HIV, or for treating infection by HIV or for treating AIDS or ARC.

8. A pharmaceutical composition useful for synergistically inhibiting HIV reverse transcriptase, comprising an effective amount of the combination as claimed in any one of Claims 1 to 5, and a pharmaceutically acceptable carrier.

9. A pharmaceutical composition useful for preventing or treating infection by HIV or for treating AIDS or ARC, comprising an effective amount of the combination as claimed in any one of Claims 1 to 5, and a pharmaceutically acceptable carrier.

10. A product containing a Class A compound as defined in Claim 1 and a Class B compound as defined in Claim 1 as a combined preparation for simultaneous, separate or sequential use in preventing or treating infection by HIV or in treating AIDS or ARC.